# FASCICULE DE BREVET EUROPEEN

(11) **EP 1 824 422 B1**
(45) Date de publication et mention de la délivrance du brevet: **07.01.2009**
(21) Numéro de dépôt: 05811145.1
(22) Date de dépôt: 08.11.2005
(51) Int. Cl.: A61F 2/34

(54) **Prothèse acétabulaire destinée à être fixée sans ciment**
Hüftgelenksprothese zur zementlosen Fixierung
Acetabular prosthesis to be fixed without cement

(30) Priorité: 08.11.2004 FR 0411865
(43) Date de publication de la demande: 29.08.2007
(73) Titulaire: CENTRE NATIONAL DE LA RECHERCHE SCIENTIFIQUE (CNRS) Etablissement Public, 75016 Paris (FR); Universite Technologie De Compiegne-UTC, 60203 Compiegne cedex (FR)
(72) Inventeur: HO BA THO, Marie-Christine, F-60200 COMPIEGNE (FR); ROUX, François, F-75006 PARIS (FR); VANLEENE, Maximilien, F-59290 WASQUEHAL (FR)
(74) Mandataire: Texier, Christian
(86) Numéro de dépôt international: PCT/EP2005/055810
(87) Numéro de publication internationale: WO 2006/048461

(56) Documents cités:
- EP-A- 0 963 740
- EP-A- 1 068 843
- WO-A-01/35873
- WO-A-96/04862
- DE-A- 3 630 276
- DE-A- 19 731 442
- DE-A- 19 915 814
- FR-A- 2 551 655
- FR-A- 2 641 461
- FR-A- 2 715 556
- FR-A- 2 788 685
- GB-A- 2 351 671
- US-B1- 6 626 949

## Description

L'invention concerne le domaine des prothèses acétabulaires destinées à être fixées sans ciment.

L'arthroplastie de la hanche est une opération chirurgicale qui consiste à remplacer l'articulation de la hanche par une articulation artificielle, appelée « prothèse totale de hanche ». La prothèse totale de hanche comprend d'une part une prothèse fémorale destinée à être fixée dans le fémur, la prothèse fémorale comprenant un tige munie d'une tête fémorale, et d'autre part une prothèse acétabulaire (ou cotyle) destinée à être fixée dans la cavité acétabulaire de l'os du bassin, la prothèse acétabulaire présentant une cavité destinée à recevoir la tête fémorale de la prothèse fémorale.

Il existe actuellement plusieurs techniques de fixation des prothèses acétabulaires.

Selon certaines techniques, la prothèse acétabulaire est fixée dans l'os grâce à un ciment, généralement à base de PolyMéthylMéthAcrylate (PMMA). Cette technique conduit à de bons résultats à moyen et long terme. Cependant, on constate de manière systématique un descellement asceptique de la prothèse à long terme.

Selon d'autres techniques, la prothèse acétabulaire est fixée dans l'os sans ciment par des moyens mécaniques. La fixation sans ciment doit permettre, dans les premiers mois après implantation, une tenue mécanique efficace de la prothèse dans l'os afin de favoriser une ostéointégration, c'est-à-dire une intégration de la prothèse aux tissus de l'os. Les prothèses sans ciment utilisées sont généralement constituées d'une cupule métallique et d'un insert en PolyEthylène (PE) disposé dans la cupule métallique. La fixation de la cupule métallique dans l'os peut être réalisée par différents moyens de fixation. Ces moyens de fixation peuvent notamment comprendre des vis, des plots, des ailettes ou des picots. En outre, la prothèse acétabulaire peut être fixée dans la cavité osseuse par insertion en force (ou press-fit) de sorte que la prothèse soit maintenue serrée dans la cavité osseuse.

Le document FR 2 641 641 (publié le 13 juillet 1990) décrit une prothèse acétabulaire utilisable sans ciment, comprenant une calotte hémisphérique munie sur son périmètre d'une série d'ailettes disposées de manière uniforme sur la surface convexe de la calotte et un élément couvrant destiné à être appliqué par enclenchement sur la surface concave de la calotte. Les ailettes présentent une forme de coins dont les sommets sont tournés vers le sommet de la calotte.

Ce document indique que les ailettes sont conçues pour être engagées par forcement dans le logement osseux, pour ainsi définir un engagement par insertion associé à un effet de compression osseuse qui dépend directement de la forme en coin des ailettes.

Les ailettes ainsi disposées agissent pour l'essentiel sur la bordure de la zone d'acétabule, c'est-à-dire là où l'os est très compact et peut donc de ce fait supporter les efforts exercés.

Le problème que se propose de résoudre l'invention est de fournir une prothèse acétabulaire destinée à être fixée dans une cavité osseuse sans ciment, présentant une bonne tenue mécanique dans la cavité osseuse tout en évitant une détérioration de l'os en contact avec la prothèse.

Ce problème est résolu dans le cadre de la présente invention grâce à une prothèse acétabulaire comprenant un insert de forme générale hémisphérique, présentant un bord et un sommet, l'insert étant muni sur sa surface extérieure d'au moins une ailette qui présente une facette s'étendant depuis le bord de l'insert et se prolongeant vers le sommet de l'insert en formant une crête, la crête présentant une forme sensiblement incurvée.

La facette de l'ailette est destinée à venir en appui sur l'os cortical sans léser le tissu osseux. La facette permet une répartition des contraintes exercées par l'insert sur l'os cortical.

La forme incurvée de la crête de l'ailette permet d'ancrer la prothèse dans l'os spongieux tout en évitant également de léser le tissu osseux.

La ou les ailette(s) assure(nt) une tenue mécanique de la prothèse dans l'os jusqu'à ce que l'ostéointégration soit suffisante pour prendre le relais. En particulier, les ailettes maintiennent l'insert en position durant un mouvement de la tête fémorale.

La prothèse pourra présenter les caractéristiques suivantes :
- la facette de l'ailette s'étend de manière adjacente à la surface extérieure de l'insert au niveau de la couronne de l'insert,
- l'ailette présente deux arêtes délimitant la facette, les deux arêtes s'étendant chacune à partir de la couronne de l'insert et se rejoignant au niveau de la crête,
- la facette présente une forme générale triangulaire,
- la facette s'étend le long d'une surface cylindrique ayant pour axe, l'axe de l'insert,
- la crête présente une forme incurvée convexe vers le sommet de l'insert,
- la crête s'étend dans un plan radial de l'insert,
- la prothèse comprend une pluralité d'ailettes identiques,
- la prothèse comprend au moins 12 ailettes,
- les ailettes sont réparties de manière uniforme autour de la couronne de l'insert,
- la couronne de l'insert présente une forme générale arrondie,
- la prothèse est formée en titane,
- la prothèse présente une surface interne concave destinée à recevoir une tête fémorale, la surface interne étant recouverte d'une couche de revêtement en DLC (Diamond Like Carbon),
- la prothèse présente une surface externe convexe destinée à être en contact avec de l'os, la surface externe étant recouverte d'une couche de revêtement en hydroxy-apatite,
- la prothèse présente une surface externe convexe destinée à être en contact avec de l'os, la surface externe présentant une rugosité comprise entre 2 µm et 4 µm, de préférence de l'ordre de 3 µm,
- la prothèse est formée en une seule pièce.

D'autres caractéristiques et avantages ressortiront encore de la description qui suit, laquelle est purement illustrative et non limitative. La description doit être lue en regard des figures annexées, qui représentent une prothèse acétabulaire conforme à un mode de réalisation possible de l'invention :
- la figure 1 représente de manière schématique, la prothèse acétabulaire, en vue de côté,
- la figure 2 représente de manière schématique, la prothèse acétabulaire, en vue de dessus,
- la figure 3 représente de manière schématique, la prothèse acétabulaire, en vue en coupe,
- la figure 4 représente de manière schématique, une ailette de la prothèse acétabulaire.

La prothèse acétabulaire représentée sur ces figures comprend un insert 1 de forme générale hémisphérique. L'insert 1 présente une surface externe convexe 2 destinée à être en contact avec l'os de la cavité acétabulaire du bassin et une surface interne concave 3 destinée à recevoir une tête fémorale.

L'insert 1 présente un sommet 4 et une couronne 5. Le « sommet » désigne le point d'intersection de l'axe X de révolution de l'insert 1 avec la surface externe 2 de l'insert. La « couronne » désigne le bord de l'insert 1 s'étendant entre la surface externe 2 et la surface interne 3.

L'insert 1 est muni sur sa surface externe 2 d'une pluralité d'ailettes 6 identiques réparties de manière uniforme autour de la couronne 5.

Chaque ailette 6 est symétrique par rapport à un plan radial de l'insert 1, c'est-à-dire un plan passant par l'axe de l'insert (noté X). Chaque ailette présente deux flancs latéraux 7 et 8. Chaque ailette 6 présente en outre une facette 9 et une crête 10 s'étendant entre les flancs latéraux 7 et 8. Les flancs latéraux 7 et 8 s'étendent de part et d'autre de la facette 9 et se rejoignent au niveau de la crête 10. Les flancs 7 et 8 forment au niveau de leur jonction avec la facette 9 des arêtes 11 et 12.

Chaque facette 9 est de forme générale triangulaire avec un sommet dirigé vers le sommet 4 de l'insert. Chaque facette 9 s'étend le long d'une surface cylindrique ayant pour axe, l'axe X de l'insert 1. La facette 9 s'étend depuis la couronne 5 de l'insert 1 et se prolonge vers le sommet 4 de l'insert en formant la crête 10. Les deux arêtes 11 et 12 délimitant la facette 9 s'étendent ainsi chacune à partir de la couronne 5 et se rejoignent au niveau de la crête 10.

Chaque crête 8 s'étend dans un plan radial de l'insert 1. Chaque crête 8 présente une forme sensiblement incurvée, convexe vers le sommet 4 de l'insert 1.

Les facettes 9 sont destinées à venir en appui contre l'os cortical de la cavité acétabulaire, c'est-à-dire la partie la plus dure de l'os qui est en contact avec la couronne 5. La forme et la disposition des facettes 9 autour de la couronne 5 permettent une bonne répartition des contraintes autour de la couronne 5, lorsque l'insert est maintenu serré dans la cavité acétabulaire.

Les crêtes 10 sont destinées à ancrer la prothèse dans l'os spongieux (ou trabéculaire) de la cavité acétabulaire, c'est-à-dire la partie la plus tendre de l'os qui est en contact avec la surface externe convexe de l'insert 1. La forme incurvée des crêtes 10 permet un ancrage de l'insert tout en évitant d'avoir recours à des éléments très incisifs (tels que des coins par exemple) qui pourraient provoquer des lésions dans l'os.

La forme particulière des ailettes 6 permet ainsi une transition progressive entre une partie d'appui de l'insert (formée par les facettes) et une partie d'ancrage (formée par les crêtes).

Par ailleurs, la couronne 5 présente une forme générale arrondie. Cette caractéristique permet de limiter les risques de luxation de l'articulation.

L'insert 1 est formé en une seule pièce, par exemple en titane.

La surface externe 2 de l'insert 1 est traitée pour présenter une rugosité de l'ordre de 3 µm et est recouverte d'une couche de revêtement en hydroxy-apatite. Ces caractéristiques ont pour but de faciliter la colonisation de la surface externe de l'insert 1 par les cellules osseuse et de favoriser ainsi l'ostéointégration de la prothèse.

Par ailleurs, la surface interne 3 de l'insert est recouverte d'une couche de revêtement en Diamond-Like-Carbon (DLC), qui est une forme amorphe du carbone contenant une grande quantité d'hybrides sp³ du carbone, ce qui lui confère des propriétés proches de celles du diamant.

Le revêtement en DLC améliore les propriétés de résistance à l'usure de la surface interne destinée à être en contact avec la tête fémorale. En outre, du fait de son inertie chimique, la biocompatibilité du revêtement en DLC est très bonne.

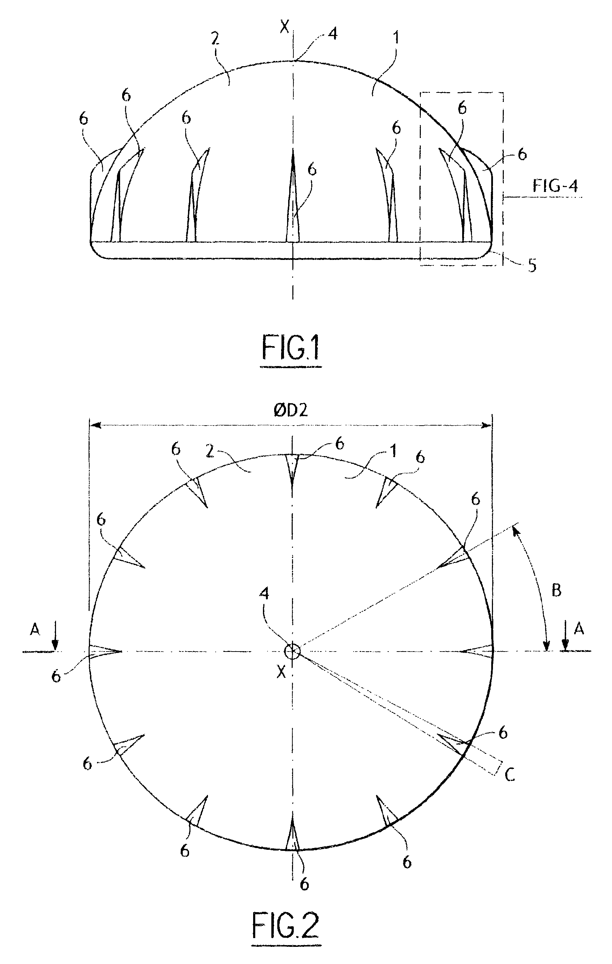

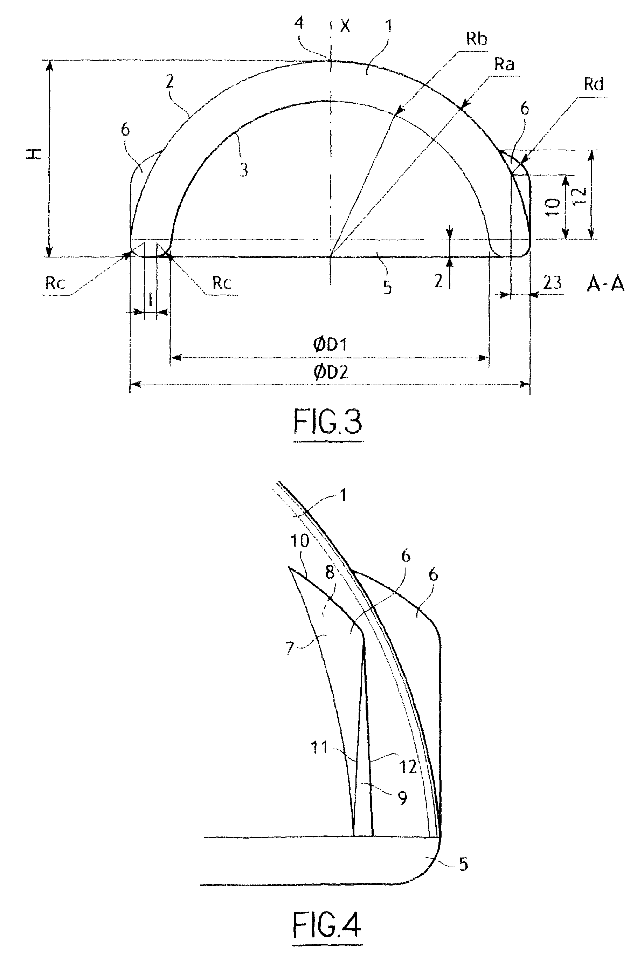

A titre d'exemple non limitatif, la prothèse représentée sur les figures 1 à 4 présente les dimensions suivantes :

| | | |
|---|---|---|
| Diamètre interne de l'insert | ∅D1 | 40 mm |
| Diamètre externe de l'insert | ∅D2 | 50 mm |
| Rayon interne de l'insert | Rb | 20 mm |
| Rayon externe de l'insert | Ra | 25 mm |
| Hauteur de l'insert | H | 25 mm |
| Rayon de la couronne | Rc | 2 mm |
| Hauteur d'une facette | | 10 mm |
| Hauteur d'une ailette | | 12 mm |
| Rayon de courbure d'une crête | Rd | 2,3 mm |
| Angle entre deux ailettes | B | 30° |
| Angle occupé par une ailette | C | 5° |

## Revendications

1. Prothèse acétabulaire comprenant un insert (1) de forme générale hémisphérique, présentant une couronne (5) et un sommet (4), l'insert (1) étant muni sur sa surface extérieure (2) d'au moins une ailette (6) qui présente une facette (9) s'étendant depuis la couronne (5) de l'insert (1) et se prolongeant vers le sommet (4) de l'insert (1) en formant une crête (10), **caractérisé en ce que** la crête (10) présente une forme sensiblement incurvée.

2. Prothèse selon la revendication 1, dans laquelle la facette (9) de l'ailette (6) s'étend de manière adjacente à la surface extérieure (2) de l'insert (1) au niveau de la couronne (5) de l'insert (1).

3. Prothèse selon l'une des revendications qui précèdent, dans laquelle l'ailette (6) présente deux arêtes (11, 12) délimitant la facette (9), les deux arêtes (11, 12) s'étendant chacune à partir de la couronne (5) de l'insert (1) et se rejoignant au niveau de la crête (10).

4. Prothèse selon l'une des revendications qui précèdent, dans laquelle la facette (9) présente une forme générale triangulaire.

5. Prothèse selon l'une des revendications qui précèdent, dans laquelle la facette (9) s'étend le long d'une surface cylindrique ayant pour axe, l'axe de l'insert (X).

6. Prothèse selon l'une des revendications qui précèdent, dans laquelle la crête (10) présente une forme incurvée convexe vers le sommet (4) de l'insert (1).

7. Prothèse selon l'une des revendications qui précèdent, dans laquelle la crête (10) s'étend dans un plan radial de l'insert (1).

8. Prothèse selon l'une des revendications qui précèdent, comprenant une pluralité d'ailettes (6) identiques.

9. Prothèse selon la revendication 8, comprenant au moins 12 ailettes (6).

10. Prothèse selon l'une des revendications 8 ou 9, dans laquelle les ailettes (6) sont réparties de manière uniforme autour de la couronne (5) de l'insert (1).

11. Prothèse selon l'une des revendications qui précèdent, dans laquelle la couronne (5) de l'insert (1) présente une forme générale arrondie.

12. Prothèse selon l'une des revendications qui précèdent, formée en titane.

13. Prothèse selon l'une des revendications qui précèdent, présentant une surface interne (3) concave destinée à recevoir une tête fémorale, la surface interne (3) étant recouverte d'une couche de revêtement en DLC (Diamond Like Carbon).

14. Prothèse selon l'une des revendications qui précèdent, présentant une surface externe (2) convexe destinée à être en contact avec de l'os, la surface externe (2) étant recouverte d'une couche de revêtement en hydroxy-apatite.

15. Prothèse selon l'une des revendications qui précèdent, présentant une surface (2) externe convexe destinée à être en contact avec de l'os, la surface externe (2) présentant une rugosité comprise entre 2 µm et 4 µm, de préférence de l'ordre de 3 µm.

16. Prothèse selon l'une des revendications qui précèdent, formée en une seule pièce.

## Claims

1. An acetabular prosthesis comprising an insert (1) with a general hemispherical shape, having a crown (5) and an apex (4), the insert (1) being provided on its outer surface (2) with at least one fin (6) which has a facet (9) extending from the crown (5) of the insert (1) and continuing towards the apex (4) of the insert (1) by forming a crest (10), **characterized in that** the crest (10) has a substantially curved shape.

2. The prosthesis according to claim 1, wherein the facet (9) of the fin (6) extends adjacent to the outer surface (2) of the insert (1) at the crown (5) of the insert (1).

3. The prosthesis according to any of the preceding claims, wherein the fin (6) has two edges (11, 12) delimiting the facet (9), both edges (11, 12) each extending from the crown (5) of the insert (1) and joining together at the crest (10).

4. The prosthesis according to any of the preceding claims, wherein the facet (9) has a general triangular shape.

5. The prosthesis according to any of the preceding claims, wherein the facet (9) extends along a cylindrical surface with as an axis, the axis of the insert (X).

6. The prosthesis according to any of the preceding claims, wherein the crest (10) has a convex shape curved towards the apex (4) of the insert (1).

7. The prosthesis according to any of the preceding claims, wherein the crest (10) extends in a radial plane of the insert (1).

8. The prosthesis according to any of the preceding claims, comprising a plurality of identical fins (6).

9. The prosthesis according to claim 8, comprising at least 12 fins (6).

10. The prosthesis according to any claims 8 or 9, wherein the fins (6) are uniformly distributed around the crown (5) of the insert (1).

11. The prosthesis according to any of the preceding claims, wherein the crown (5) of the insert (1) has a general rounded shape.

12. The prosthesis according to any of the preceding claims, formed in titanium.

13. The prosthesis according to any of the preceding claims, having an internal concave surface (3) intended to receive a femoral head, the internal surface (3) being covered with a layer of Diamond-Like-Carbon (DLC) coating.

14. The prosthesis according to any of the preceding claims, having an external convex surface (2) intended to be in contact with the bone, the external surface (2) being covered with a layer of hydroxyapatite coating.

15. The prosthesis according to any of the preceding claims, having an external convex surface (2) intended to be in contact with the bone, the external surface (2) having a roughness between 2 µm and 4 µm, preferably of the order of 3 µm.

16. The prosthesis according to any of the preceding claims, formed as a single part.

## Patentansprüche

1. Hüftgelenksprothese, einen allgemein halbkugelförmigen Einsatz (1) umfassend, der einen Kranz (5) und eine Spitze (4) aufweist, wobei der Einsatz (1) auf seiner Außenfläche (2) mindestens mit einem Flügel (6) ausgestattet ist, der eine Facette (9) aufweist, die sich ab dem Kranz (5) des Einsatzes (1) erstreckt und sich in Richtung Spitze (4) des Einsatzes (1) verlängert und dabei einen Grat (10) bildet, **dadurch gekennzeichnet, dass** der Grat (10) eine deutlich gebogene Form hat.

2. Prothese nach Anspruch 1, wobei sich die Facette (9) des Flügels (6) neben der Außenfläche (2) des Einsatzes (1) auf Ebene des Kranzes (5) des Einsatzes (1) erstreckt.

3. Prothese nach einem der vorangehenden Ansprüche, wobei der Flügel (6) zwei Kanten (11, 12) aufweist, die die Facette (9) begrenzen, wobei sich die zwei Kanten (11, 12) jeweils ab dem Kranz (5) des Einsatzes (1) erstrecken und sich auf Ebene des Grats (10) begegnen.

4. Prothese nach einem der vorangehenden Ansprüche, wobei die Facette (9) eine allgemein dreieckige Form aufweist.

5. Prothese nach einem der vorangehenden Ansprüche, wobei sich die Facette (9) entlang einer zylindrischen Fläche erstreckt, deren Achse die Achse (X) des Einsatzes ist.

6. Prothese nach einem der vorangehenden Ansprüche, wobei der Grat (10) eine in Richtung Spitze (4) des Einsatzes (1) konvex gebogene Form hat.

7. Prothese nach einem der vorangehenden Ansprüche, wobei sich der Grat (10) in einer radialen Ebene des Einsatzes (1) erstreckt.

8. Prothese nach einem der vorangehenden Ansprüche, die eine Vielzahl identischer Flügel (6) umfasst.

9. Prothese nach Anspruch 8, die mindestens zwölf Flügel (6) umfasst.

10. Prothese nach einem der Ansprüche 8 oder 9, wobei die Flügel (6) gleichmäßig um den Kranz (5) des Einsatzes (1) verteilt sind.

11. Prothese nach einem der vorangehenden Ansprüche, wobei der Kranz (5) des Einsatzes (1) eine allgemein abgerundete Form aufweist.

12. Prothese nach einem der vorangehenden Ansprüche, die aus Titan gebildet ist.

13. Prothese nach einem der vorangehenden Ansprüche, die eine konkave Innenfläche (3) aufweist, die zur Aufnahme eines Oberschenkelkopfs bestimmt ist, wobei die Innenfläche (3) mit einer Schicht aus DLC (Diamond Like Carbon) beschichtet ist.

14. Prothese nach einem der vorangehenden Ansprüche, die eine konvexe Außenfläche (2) aufweist, die zur Berührung mit dem Knochen bestimmt ist, wobei die Außenfläche (2) mit einer Hydroxyapatitschicht beschichtet ist.

15. Prothese nach einem der vorangehenden Ansprüche, die eine konvexe Außenfläche (2) aufweist, die zur Berührung mit dem Knochen bestimmt ist, wobei die Außenfläche (2) eine Körnung zwischen 2 µm und 4 µm inklusive, vorzugsweise in der Größenordnung von 3 µm, aufweist.

16. Prothese nach einem der vorangehenden Ansprüche, die aus einem einzigen Teil gebildet ist.
